# EUROPEAN PATENT APPLICATION

(11) **EP 4 767 998 A2**
(43) Date of publication of application: **01.07.2026**
(21) Application number: 26178158.7
(22) Date of filing: 10.11.2022
(51) Int. Cl.: A61F 2/46

(54) **MODULAR STEMLESS IMPLANTS FOR ARTHROPLASTY IMPLANT SYSTEMS**

(30) Priority: 11.11.2021 US 202163278232 P
(62) Divisional of application: 22823264.1
(71) Applicant: Arthrex, Inc., Naples, FL 34108-1945 (US)
(72) Inventor: Paterson, John David, Naples, FL, 34109 (US); Knight, Michael Alan, Naples, FL, 34109 (US); Denard, Patrick J., Jacksonville, OR, 97530 (US)
(74) Representative: Lohr, Jöstingmeier & Partner Patent- und Rechtsanwälte mbB

(57) **Abstract**

Arthroplasty implant systems and methods are provided for restoring the functionality of a joint. The arthroplasty implant systems may include a modular threaded cup having a cylindrical shaped body and a flange that is removably connectable to the cylindrical shaped body. A thread may be provided on the cylindrical shaped body. The thread is configured to engage a cortical and/or cancellous bone of a bone, and the flange is sized to engage a cortical rim of the bone.

## Description

### BACKGROUND

This disclosure relates to the field of arthroplasty, and more particularly to arthroplasty implant systems that include modular implants capable of establishing a stemless convertible platform for interfacing with articular implants.

Many bones of the human musculoskeletal system include articular surfaces. The articular surfaces cooperate to facilitate different types and degrees of joint movement. The articular surfaces can erode or experience bone loss over time due to repeated use or wear, thereby causing joint instability and pain.

Arthroplasty is an orthopedic surgical procedure performed to repair or replace joints that exhibit degenerative bone deficiencies. Bone deficiencies may occur along the articular surfaces of bone. Some arthroplasty procedures utilize one or more implants to repair the articular surfaces.

US 2019/0175354 Al discloses stemless prosthesis anchor components, methods, and kits.

### SUMMARY

This disclosure relates to arthroplasty implant systems and methods designed for restoring functionality to a joint. The arthroplasty implant systems may include an implant assembly that includes a stemless convertible implant.

An exemplary humeral implant assembly for an arthroplasty implant system may include, *inter alia,* an articular implant, and a modular stemless implant adapted to establish a convertible platform for receiving the articular implant. The modular stemless implant includes a thread configured to engage cortical and/or cancellous bone of a bone and a flange sized to engage a cortical rim of the bone.

In a further embodiment, the articular implant is an anatomic articular implant that includes a convex articular surface.

In a further embodiment, the articular implant is a reverse articular implant that includes a concave articular surface.

In a further embodiment, the articular implant includes a spacer that is coupled to the stemless implant and a liner that is coupled to the spacer. The liner includes a concave articular surface.

In a further embodiment, the spacer is coupled to the stemless implant by a C-ring.

In a further embodiment, the stemless implant is comprised of a polyether ether ketone (PEEK) material

In a further embodiment, the modular stemless implant includes a receiving cavity adapted to receive the articular implant, and the receiving cavity extends inwardly from a rim to a floor of a rounded base of the modular stemless implant.

In a further embodiment, the thread is circumferentially disposed about a radially outer surface of a cylindrical shaped body of the modular stemless implant.

In a further embodiment, the flange is removably connectable to the cylindrical shaped body.

In a further embodiment, an outer diameter of the flange is greater than an outer diameter of the cylindrical shaped body at a tip of the thread.

In a further embodiment, the cylindrical shaped body includes a plurality of pockets adapted to facilitate bony ingrowth.

In a further embodiment, the flange includes a plurality of suture eyelets that are each configured to receive a thread-like material.

An exemplary arthroplasty implant system may include, *inter alia,* a two-piece modular threaded cup having a cylindrical shaped body and a flange that is removably connectable to the cylindrical shaped body. A thread may be provided on the cylindrical shaped body. The thread is configured to engage cortical and/or cancellous bone of a bone, and the flange is configured to engage a cortical rim of the bone.

In a further embodiment, the flange is a separate component from the cylindrical shaped body to establish the two-piece modular design of the threaded cup.

In a further embodiment, the threaded cup embodies an inlay design that establishes a convertible platform for receiving an articular implant.

In a further embodiment, the flange is provided on a first side of the cylindrical shaped body and a rounded base is provided on a second side of the cylindrical shaped body.

In a further embodiment, a receiving cavity extends inwardly from a rim of the cylindrical shaped body to a floor of the rounded base, and the floor establishes an inner surface of the rounded base.

In a further embodiment, at least one engagement opening is formed through the rounded base.

In a further embodiment, an outer diameter of the flange is greater than an outer diameter of the cylindrical shaped body at a tip of the thread.

In a further embodiment, the cylindrical shaped body includes a plurality of pockets adapted to facilitate bony ingrowth.

In a further embodiment, the flange includes a plurality of suture eyelets that are each configured to receive a thread-like material.

In a further embodiment, an inserter system includes a drive shaft configured to engage the cylindrical shaped body of the threaded cup and a cage assembly configured to engage the flange of the threaded cup.

In a further embodiment, the drive shaft includes an inner shaft having a threaded distal tip for engaging an engagement opening of the cylindrical shaped body, and the cage assembly includes a mounting leg having a tapered tooth configured to engage a tapered slot of the flange.

An exemplary surgical method may include, inter alia, preparing a bone for receiving a threaded cup, positioning a flange of the threaded cup against a cortical rim of the bone, and screwing a cylindrical shaped body of the threaded cup through the flange and into the bone.

### Clauses:

1. A humeral implant assembly for an arthroplasty implant system, comprising:
   an articular implant; and a modular stemless implant adapted to establish a convertible platform for receiving the articular implant; wherein the modular stemless implant includes a thread configured to engage a bone and a flange sized to engage a cortical rim of the bone.
2. The assembly as recited in clause 1, wherein the articular implant includes a spacer that is coupled to the stemless implant and a liner that is coupled to the spacer, and further wherein the liner includes a concave articular surface, and optionally wherein the spacer is coupled to the stemless implant by a C-clip.
3. The assembly as recited in clause 1 or 2, wherein the modular stemless implant is comprised of a polyether ether ketone (PEEK) material.
4. The assembly as recited in any preceding clause, wherein the modular stemless implant includes a receiving cavity adapted to receive the articular implant, and further wherein the receiving cavity extends inwardly from a rim to a floor of a rounded base of the modular stemless implant.
5. The assembly as recited in any preceding clause, wherein the thread is circumferentially disposed about a radially outer surface of a cylindrical shaped body of the modular stemless implant, and optionally wherein the flange is removably connectable to the cylindrical shaped body.
6. The assembly as recited in clause 5, wherein an outer diameter of the flange is greater than an outer diameter of the cylindrical shaped body at a tip of the thread, and optionally wherein the cylindrical shaped body includes a plurality of pockets adapted to facilitate bony ingrowth.
7. The assembly as recited in any preceding clause, wherein the flange includes a plurality of suture eyelets that are each configured to receive a thread-like material.
8. An arthroplasty implant system, comprising: a two-piece modular threaded cup that includes a cylindrical shaped body, a thread provided on the cylindrical shaped body, and a flange removably connectable to the cylindrical shaped body, wherein the thread is configured to engage a bone and the flange is sized to engage a cortical rim of the bone.
9. The system as recited in clause 8, wherein the flange is a separate component from the cylindrical shaped body to establish the two-piece modular design of the threaded cup.
10. The system as recited in clause 8 or 9, wherein the threaded cup embodies an inlay design that establishes a convertible platform for receiving an articular implant.
11. The system as recited in any of clauses 8 to 10, wherein the flange is provided on a first side of the cylindrical shaped body and a rounded base is provided on a second side of the cylindrical shaped body.
12. The system as recited in clause 11, wherein a receiving cavity extends inwardly from a rim of the cylindrical shaped body to a floor of the rounded base, and further wherein the floor establishes an inner surface of the rounded base, and optionally comprising at least one engagement opening formed through the rounded base.
13. The system as recited in any of clauses 8 to 12, wherein an outer diameter of the flange is greater than an outer diameter of the cylindrical shaped body at a tip of the thread.
14. The system as recited in any of clauses 8 to 13, comprising an inserter system that includes a drive shaft configured to engage the cylindrical shaped body and a cage assembly configured to engage the flange, and optionally wherein the drive shaft includes an inner shaft having a threaded distal tip for engaging an engagement opening of the cylindrical shaped body, and the cage assembly includes a mounting leg having a tapered tooth configured to engage a tapered slot of the flange.
15. A surgical method, comprising: preparing a bone for receiving the threaded cup of clause 8; positioning the flange against a cortical rim of the bone; and screwing the cylindrical shaped body through the flange and into the bone.

### BRIEF DESCRIPTION OF THE DRAWINGS

Figure 1 illustrates a humeral implant assembly of an arthroplasty implant system.
Figure 2 illustrates another exemplary humeral implant assembly of an arthroplasty implant system.
Figure 3 is an exploded view of an exemplary threaded cup of a humeral implant assembly.
Figure 4 is a cross-sectional perspective view of the threaded cup of Figure 3.
Figure 5 illustrates multiple flange options that may be provided for the threaded cup of Figure 3.
Figure 6 illustrates a flange of the threaded cup of Figure 3 engaging a cortical rim of a bone.
Figure 7 illustrates various details associated with a circumferential thread of the threaded cup of Figures 3-6.
Figure 8 schematically illustrates an exemplary surgical method for performing a shoulder arthroplasty procedure.
Figure 9 illustrates a humeral implant assembly that includes a threaded cup.
Figure 10 is an exploded view of the humeral implant assembly of Figure 9.
Figure 11 is a cross-sectional view of the humeral implant assembly of Figure 9.
Figures 12A and 12B illustrate a spacer of another exemplary humeral implant assembly.
Figure 13 illustrates a liner of the humeral implant assembly of Figure 9.
Figure 14 illustrates another exemplary liner of the humeral implant assembly of Figure 9.
Figure 15 illustrates an inserter system for implanting a threaded cup.
Figure 16 is an exploded view of the inserter system of Figure 15.
Figure 17 is a cross-sectional view of the inserter system of Figure 15.
Figure 18 illustrates select portion of the inserter system of Figure 15.
Figure 19 illustrates additional select portions of the inserter system of Figure 15.

### DETAILED DESCRIPTION

This disclosure describes arthroplasty implant systems and methods for restoring the functionality of a joint. The arthroplasty implant systems may include implants capable of establishing a stemless convertible platform for interfacing with articular implants.

In some implementations, the arthroplasty implant systems of this disclosure may include a modular threaded cup having a cylindrical shaped body and a flange that is removably connectable to the cylindrical shaped body. A thread may be provided on the cylindrical shaped body. The thread is configured to engage cortical and/or cancellous bone of a bone, and the flange is sized to engage a cortical rim of the bone. These and other features of this disclosure are further detailed below.

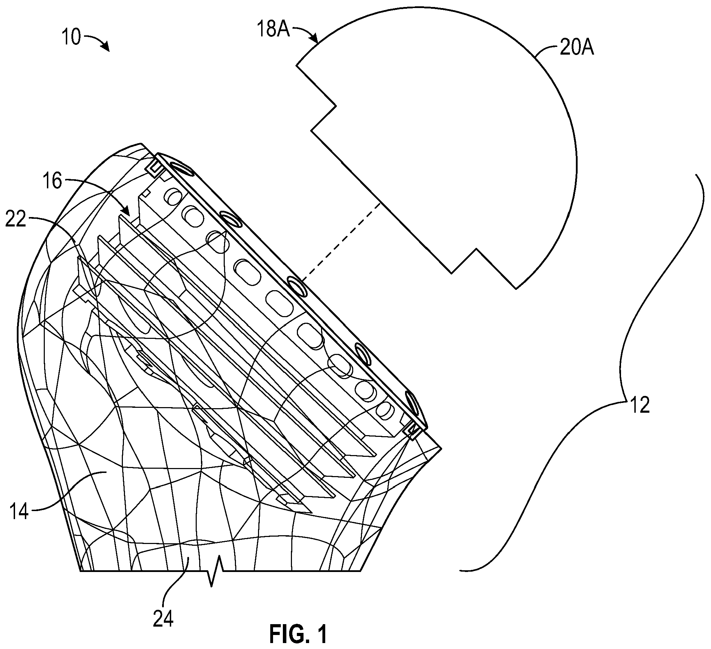

Figure 1 illustrates an arthroplasty implant system 10 that includes a humeral implant assembly 12. The humeral implant assembly 12 may be implanted within a humerus 14 of a shoulder joint to aid in reconstructing the native articular surface of the humerus 14 and/or restoring the functionality (e.g., range or motion, stability, etc.) of the shoulder joint. Although not shown, the arthroplasty implant system 10 could additionally include a glenoid implant assembly that is configured to interface with the humeral implant assembly 12 for restoring functionality to the shoulder joint. Moreover, although the teachings of this disclosure are described with specific reference to the shoulder joint, this disclosure is not intended to be limited to any particular joint of the human musculoskeletal system and could be applicable to other joints, such as the hip joint, for example.

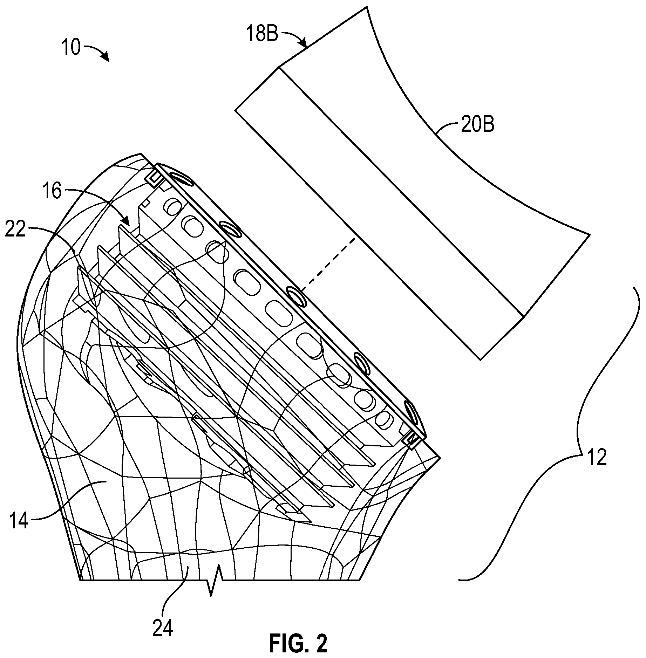

The humeral implant assembly 12 may include a threaded cup 16 and an anatomic articular implant 18A (see Figure 1) or a reverse articular implant 18B (see Figure 2). The anatomic articular implant 18A may be utilized in combination with the threaded cup 16 for performing anatomic total shoulder arthroplasty procedures, and the reverse articular implant 18B may be utilized in combination with the threaded cup 16 for performing reverse shoulder arthroplasty procedures. In reverse shoulder arthroplasty procedures, the reconstituted humerus 14 provides the socket portion and the glenoid (not shown) provides the ball portion of the ball-and-socket joint, which is the opposite of the native anatomy. The anatomic articular implant 18A may include a convex articular surface 20A, and the reverse articular implant 18B may include a concave articular surface 20B. The articular surfaces 20A, 20B are configured to interface with the native glenoid or a glenoid implant assembly of the arthroplasty implant system 10.

In some implementations, the threaded cup 16, the anatomic articular implant 18A, and the reverse articular implant 18B may be provided together as part of a surgical kit. The surgical kit could additionally could multiple sizes of each of the threaded cup 16, the anatomic articular implant 18A, and the reverse articular implant 18B.

In the illustrated embodiment, a humeral head of the humerus 14 has been resected, and thus the native articular component of the humerus 14 is removed in order to prepare the humerus 14 for receiving the humeral implant assembly 12. After the humerus 14 has been appropriately prepared, the threaded cup 16 may be screwed into a metaphysis 22 of the humerus 14. In an embodiment, the threaded cup 16 is a stemless implant of the humeral implant assembly 12 and therefore lacks a stem that extends into a diaphysis 24 of the humerus 14. The threaded cup 16 may be configured to establish a convertible platform for receiving either the anatomic articular implant 18A or the reverse articular implant 18B. The anatomic articular implant 18A or the reverse articular implant 18B may be mounted to the threaded cup 16 for assembling the humeral implant assembly 12.

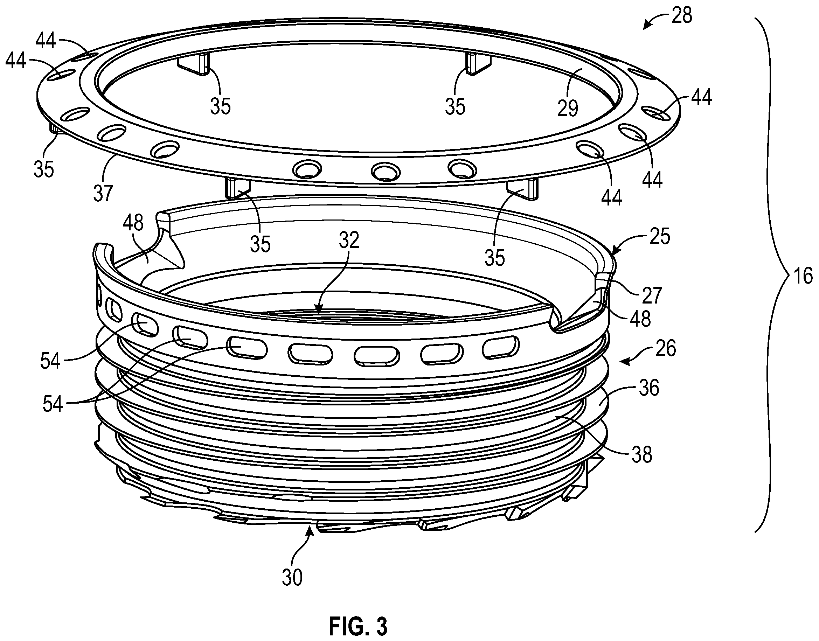

The threaded cup 16 of the humeral implant assembly 12 is further illustrated in Figures 3, 4, and 5 (with continued reference to Figures 1 and 2). In an embodiment, the threaded cup 16 is constructed of a titanium material. In another embodiment, the threaded cup 16 may be made of a material that has an elastic modulus that is relatively close to that of cortical bone, such as polyether ether ketone (PEEK), for example. Other materials for constructing the threaded cup 16 could alternatively be utilized within the scope of this disclosure.

The threaded cup 16 may include a cylindrical shaped body 26 and a flange 28. In this embodiment, the threaded cup 16 embodies a two-piece modular design in which the flange 28 is removably connectable to the cylindrical shaped body 26.

The cylindrical shaped body 26 may extend between a rim 25 located at a top or proximal side of the cylindrical shaped body 26 and a rounded base 30 located at a bottom or distal side of the cylindrical shaped body 26. The rim 25 may thus be located on an opposite side of the cylindrical shaped body 26 from the rounded base 30.

The flange 28 may be removably connected to the rim 25 of the cylindrical shaped body 26. For example, the rim 25 of the cylindrical shaped body 26 may include a tapered outer circumferential surface 27, and the flange 28 may include a tapered inner circumferential surface 29. The tapered inner circumferential surface 29 may be configured to engage the tapered outer circumferential surface 27 via an interference fit to connect the flange 28 to the cylindrical shaped body 26. In an embodiment, the tapered outer circumferential surface 27 and the tapered inner circumferential surface 29 taper in a proximal-to-distal direction.

A receiving cavity 32 of the threaded cup 16 may be configured to receive and secure either the anatomic articular implant 18A or the reverse articular implant 18B to the threaded cup 16. The receiving cavity 32 may be circumscribed by the flange 28 and the cylindrical shaped body 26. The receiving cavity 32 may extend inwardly from the rim 25 to a floor 34 of the rounded base 30. The floor 34 may establish an inner surface of the rounded base 30.

The receiving cavity 32 may provide an inlay design in which a majority of the threaded cup 16 (with the exception of the flange 28) is disposed inside the humerus 14 post implantation. In this way, the connection between the threaded cup 16 and the articular implant 18A, 18B is also inlaid rather than exhibiting an onlay design.

A thread 36 may be circumferentially disposed about a radially outer surface 38 of the cylindrical shaped body 26. The thread 36 may be a self-tapping thread configured to allow the threaded cup 16 to be screwed into the humerus 14. The thread 36 may be configured such that either a clockwise rotation or a counterclockwise rotation functions to advance the threaded cup 16 into the humerus 14.

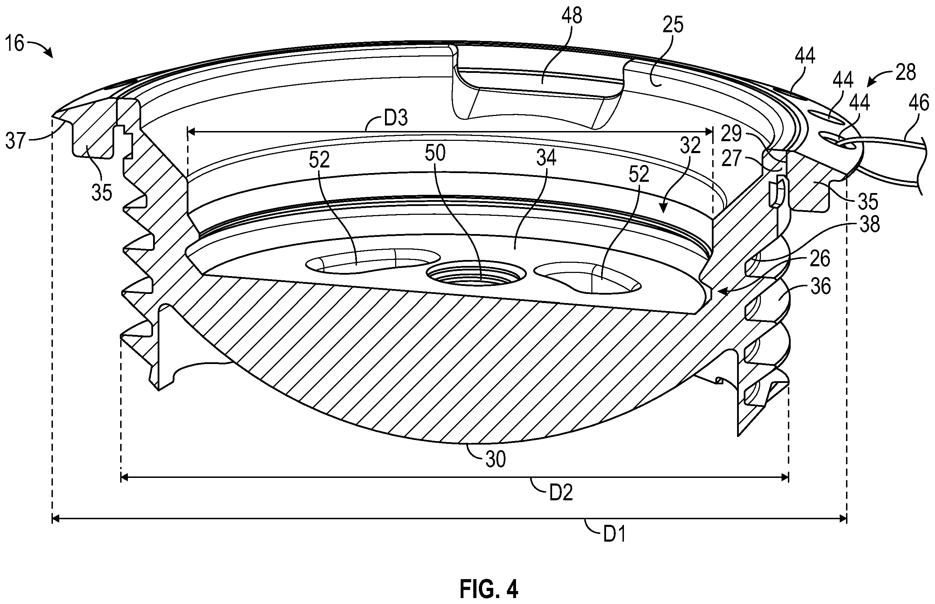

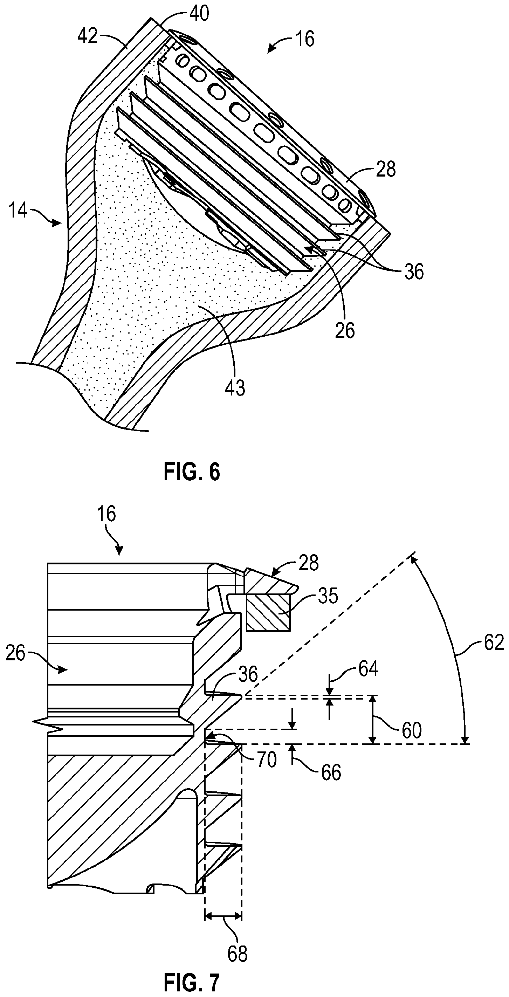

In an embodiment, an outer diameter D1 of the flange 28 is greater than an outer diameter D2 (defined here at a tip of the thread 36) of the cylindrical shaped body 26 (see, e.g., Figure 4). The outer diameter D1 of the flange 28 may be sized to enable the flange 28 to engage a cortical rim 40 (see, e.g., Figure 6) of the humerus 14. Engaging the cortical rim 40 in this manner may provide additional fixation support and load the proximal portion of the humerus 14 more favorably.

Moreover, the outer diameter D2 may be sized such that the thread 36 is positioned in relatively close proximity to cortical bone 42 of the humerus 14 once the threaded cup 16 is inserted therein (see, e.g., Figure 6). Fixating the threaded cup 16 with the thread 36 as close to the cortical bone 42 as possible may provide improved initial and long-term fixation as compared to "press-fit" implant designs. The thread 36 may thus engage cortical bone 42, cancellous bone 43, or both.

The cylindrical shaped body 26 may further include an inner diameter D3. The inner diameter D3 may establish a cup size of the threaded cup 16. The inner diameter D3 may be less than both the outer diameter D1 and the outer diameter D3.

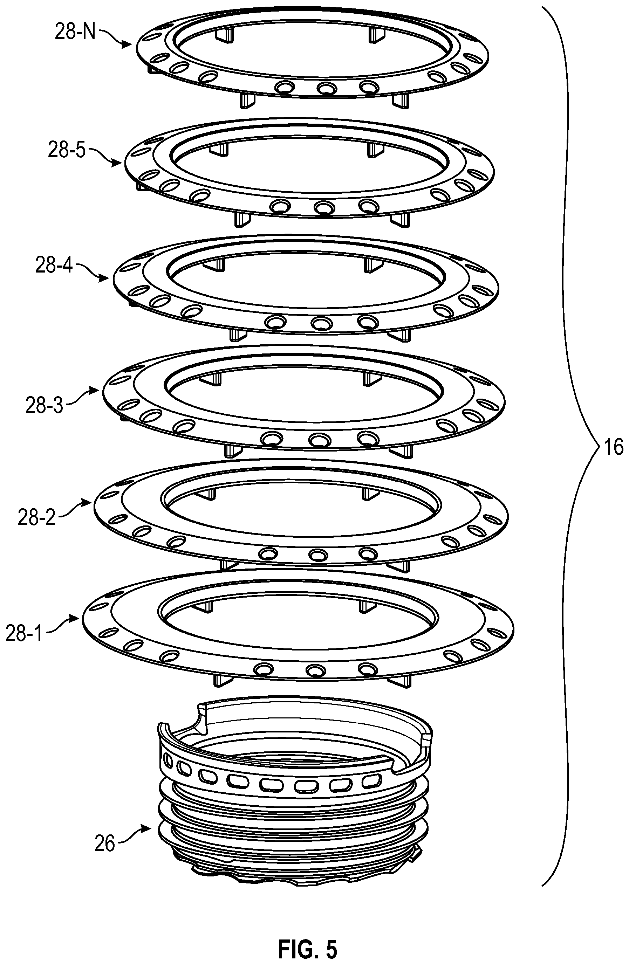

The actual dimensions of the outer diameter D1 of the flange 28, the outer diameter D2 of the cylindrical shaped body 26, and the inner diameter D3 of the cylindrical shaped body 26 may vary depending on the size of the patient, among other factors. The surgical kit referenced above could include threaded cups having multiple combinations of flange 28 outer diameter sizes and cylindrical shaped body 26 inner diameter sizes. Multiple exemplary sizes of flanges (identified as 28-1 to 28-N, where "N" is any number) that could optionally be utilized with the cylindrical shaped body 26 of the threaded cup 16 are illustrated in Figure 5.

Table 1 below illustrates exemplary sizes of threaded cups 16 that could be provided as part of the surgical kit. The listed sizes are exemplary only and thus intended to be non-limiting.

**TABLE 1 - Exemplary Sizes of Threaded Cup 16**

| | **ID of Threaded Cup 16** | 30 mm | 33 mm | 36 mm | 39 mm | 42 mm |
|---|---|---|---|---|---|---|
| **OD of Flange 28** | | | | | | |
| 41 mm | | ● | | | | |
| 43 mm | | ● | | | | |
| 45 mm | | ● | ● | | | |
| 47 mm | ● | ● | ● | | | |
| 49 mm | ● | ● | ● | | | |
| 51 mm | ● | ● | ● | ● | | |
| 53 mm | ● | ● | ● | ● | | |
| 55 mm | ● | ● | ● | ● | ● | |

The flange 28 of the threaded cup 16 may be either circular or elliptical shaped. However, the actual shape of the flange 28 is not intended to limit this disclosure.

The flange 28 may include a plurality of anti-rotation tabs 35. The anti-rotation tabs 35 may protrude from a distal-facing surface 37 of the flange 28. The anti-rotation tabs 35 may be impacted into bone (e.g., the humerus 14) for preventing the flange 28 from rotating within the bone.

A plurality of suture eyelets 44 may extend through the flange 28. The suture eyelets 44 may be configured to receive a thread-like material, such as a suture 46 (see Figure 4). The suture 46 may then be utilized to assist with tying tissue (e.g., subscapularis muscle, supraspinatus muscle, etc.) to the humerus 14 in the area around the flange 28.

One or more cutouts or interruptions 48 may be formed in the rim 25 of the cylindrical shaped body 26 of the threaded cup 16. The interruptions 48 are sized to receive mating features of a wedge/spacer (not shown) that may be utilized in combination with the threaded cup 16 for reducing laxity between the threaded cup 16 and the articular implant 18A, 18B. The mating features of the wedge/spacer may engage walls of the rim 25 that delineate the interruptions 48 to prevent rotation of the wedge/spacer relative to the threaded cup 16.

One or more engagement openings 50 may be formed through the rounded base 30 of the threaded cup 16. The engagement openings 50 may be configured to receive additional mating features of the wedge/spacer. The engagement openings 50 may be threaded round openings, for example.

The rounded base 30 may include one or more additional engagement openings 52 formed therethrough. The engagement openings 52 may be configured to receive mating features of an inserter device that can be utilized to implant the threaded cup 16 within the humerus 14. The engagement openings 52 may be oblong or round, for example.

The threaded cup 16 may additionally be equipped with a plurality of pockets 54. In some implementations, the pockets 54 may be formed in the radially outer surface 38 of the cylindrical shaped body 26 at a location just inward or distal of the rim 25. The pockets 54 may facilitate bony ingrowth post-insertion. Alternatively or additionally, a porous coating could be applied to select portions of the cylindrical shaped body 26 for facilitating bony ingrowth. In yet another embodiment, a surface finish of the thread 36 may be grit blasted to promote bony ingrowth. The pockets 54 may be oblong shaped, in an embodiment.

Figure 7 illustrates additional details associated with the thread 36 of the threaded cup 16. The thread 36 may include design characteristics such as a thread pitch 60, a thread angle 62, a thread tip width 64, a thread root width 66, a thread depth 68, and a thread root radius 70. Table 2, provided below, illustrates exemplary design characteristics of the thread 36. The disclosed design characteristics are intended to be exemplary only, and thus other thread specific formulations are contemplated as within the scope of this disclosure. In this disclosure, the term "about" means that the expressed quantities or ranges need not be exact but may be approximated and/or larger or smaller, reflecting acceptable tolerances, conversion factors, measurement error, etc.

**TABLE 2 - Exemplary Design Characteristics of Thread 36**

| **Thread Property** | **Dimension** |
|---|---|
| Thread Pitch | About 2.70 mm |
| Thread Angle | About 40° |
| Thread Tip Width | About 0.19 mm |
| Thread Root Width | About 0.83 mm |
| Thread Depth | About 2 mm |
| Thread Root Radius | About 0.20 mm |

The thread pitch 60 may be a variable pitch. In an embodiment, the variable pitch increases in a direction that extends from the proximal side of the threaded cup 16 toward the distal side of the threaded cup 16. In another embodiment, the variable pitch increases in a direction that extends from the distal side of the threaded cup toward the proximal side of the threaded cup 16

The thread 36 may be a single lead thread, a double lead thread, or a triple lead thread. The thread lead may be modified to control the amount of turns it takes to seat the threaded cup 16 within bone

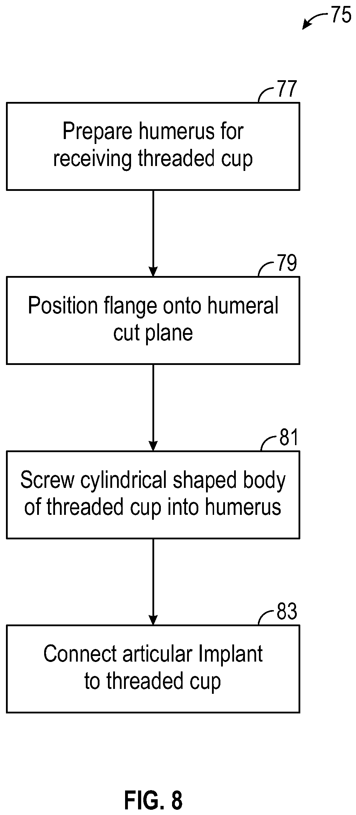

Figure 8, with continued reference to Figures 1-7, schematically illustrates a surgical method 75 for performing a shoulder arthroplasty procedure. The surgical method 75 may include implanting the threaded cup 16 during the shoulder arthroplasty procedure. However, other joints could be repaired or replaced using a similar procedure to the one described below. It should further be understood that the surgical method 75 could include a greater or fewer number of steps, and that the steps could be performed in a different order within the scope of this disclosure.

The method may begin at block 77 by preparing the humerus 14 for receiving the threaded cup 16. Preparing the humerus 14 may include resecting the humeral head of the humerus 14, preparing a cavity within the resected humerus for receiving the cylindrical shaped body 26, etc.

Next, at block 79, the flange 28 may be positioned onto the humeral cut plate. The cylindrical shaped body 26 of the threaded cup 16 may be then be screwed into the prepared humerus 14 at block 81. The cylindrical shaped body 26 may be screwed through the opening of the flange 28 and down into the humerus 14 until the rim 25 sits just proud of the cortical rim 40.

Once implantation is complete, the thread 36 of the threaded cup 16 may engage the humerus 14 near the cortical bone 42, and the flange 28 may load against the cortical rim 40. The thread 36 may therefore engage the cortical bone 42, cancellous bone 43, or both.

Finally, at block 83, an articular implant 18A, 18B may be connected to the implanted threaded cup 16. The threaded cup 16 therefore establishes a convertible platform for interfacing with either anatomic articular implants or reverse articular implants.

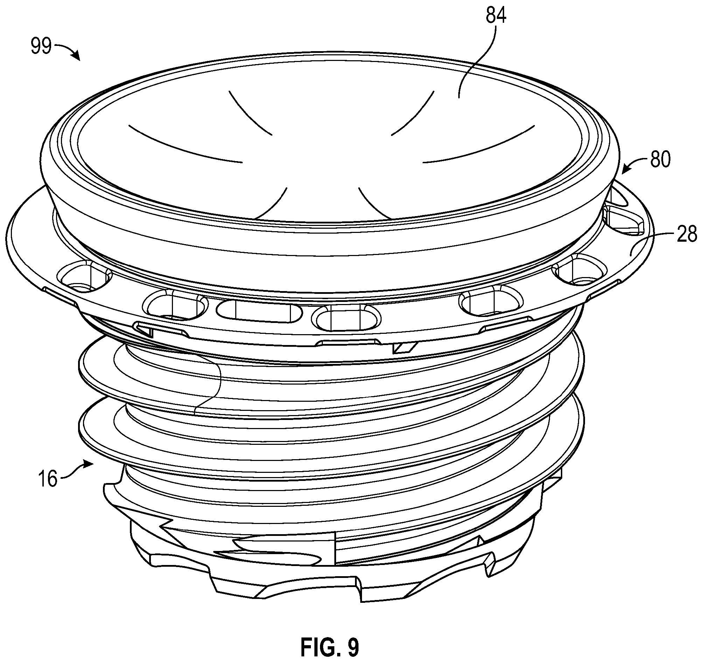

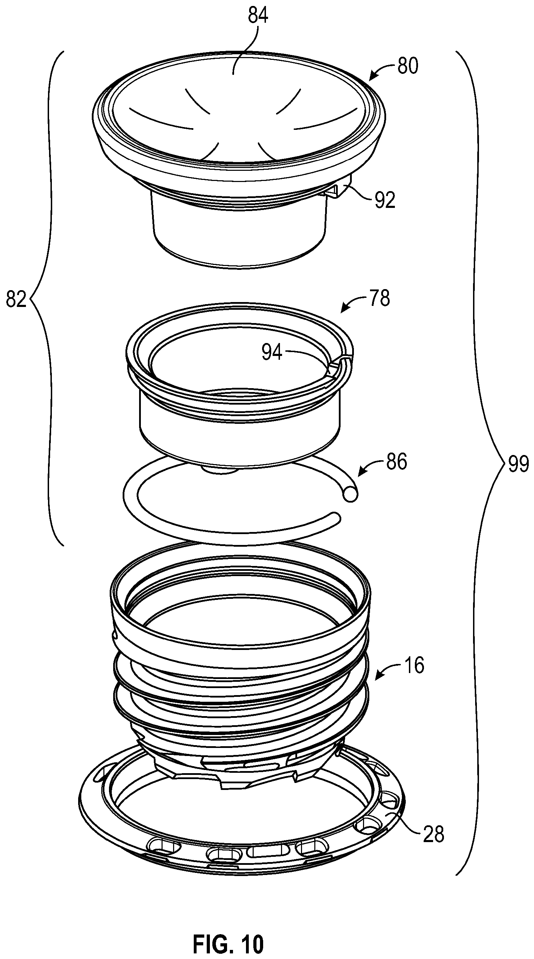

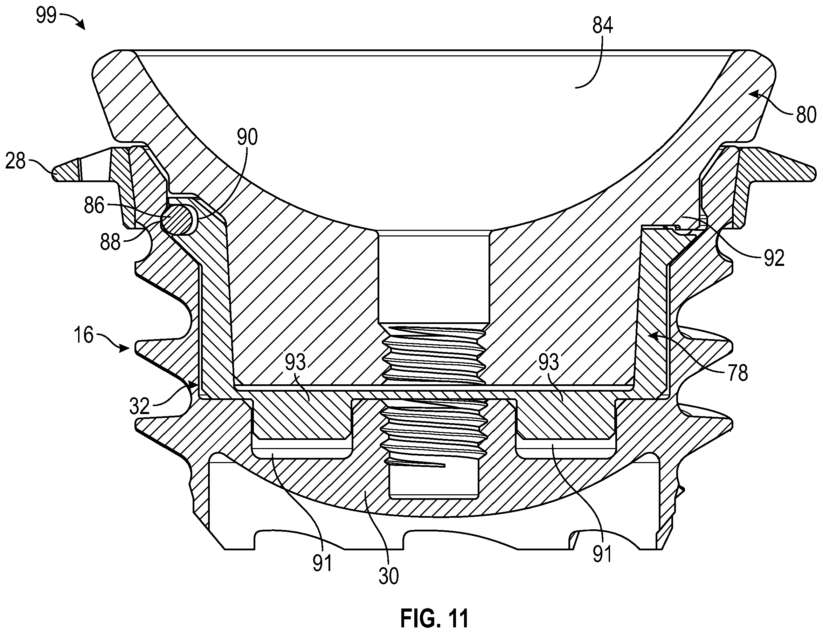

Referring now to Figures 9, 10, and 11, the threaded cup 16 may be utilized as part of a humeral implant assembly 99 of an arthroplasty implant system. In addition to the threaded cup 16, the humeral implant assembly 99 may include a spacer 78 and a liner 80. Together, the spacer 78 and the liner 80 may establish an articular implant 82 of the humeral implant assembly 99.

In this embodiment, the articular implant 82 is a reverse articular implant. Therefore, the liner 80 may include a concave articular surface 84. However, anatomic articular implants are also contemplated within the scope of this disclosure (see, e.g., Figure 1).

The threaded cup 16 may include one or more engagement openings 91 formed in the rounded base 30. The engagement openings 91 may accommodate de-rotation pegs 93 of the spacer 78 (or of the liner 80 if the spacer 78 is not used) for rotationally stabilizing the spacer 78 relative to the threaded cup 16.

A C-clip 86 may be used to couple the spacer 78 to the threaded cup 16, and the liner 80 may be coupled to the spacer 78 by a taper connection or any other connection. The C-clip 86 may be accommodated within a circumferential groove 88 formed in the receiving portion 32 of the threaded cup 16, and the C-clip 86 may be further accommodated within a circumferential groove 90 formed in the spacer 78.

In an embodiment, the liner 80 includes a lock block 92. The lock block 92 may be accommodated within a notch 94 formed in the spacer 78. The lock block 92 is configured to prevent the C-clip 86 from deforming inwards and allowing the spacer 78 to disengage from the threaded cup 16.

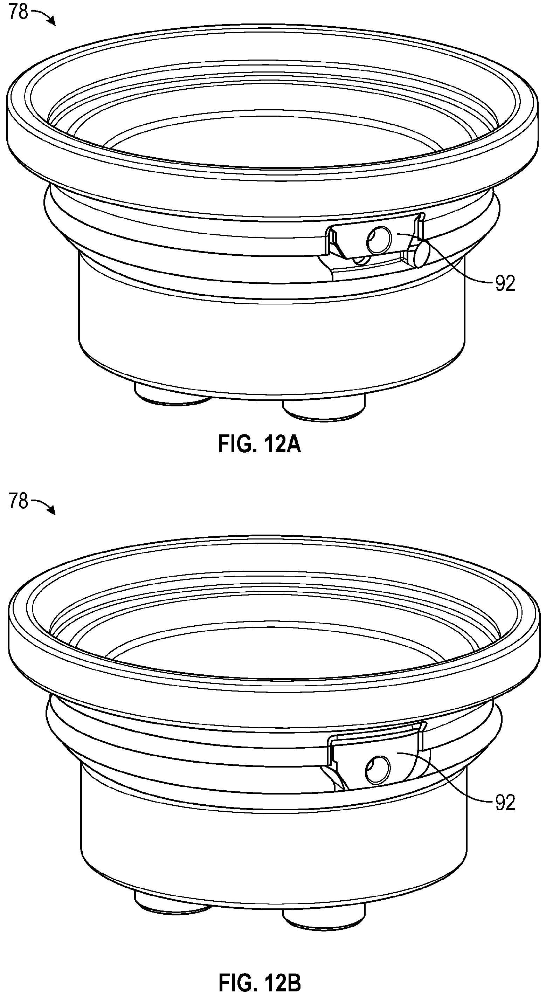

In another embodiment, the lock block 92 is provided by the spacer 78 (see, e.g., Figures 12A and 12B). In this implementation, the lock block 92 may be translated between an open position (Figure 12A) in which the C-clip 86 is free to deform, and a locked position (Figure 12B) in which the C-clip 86 is prevented from deforming. The lock block 92 may move from the open position to the locked position in response to a force applied by the liner 80 as the liner is moved into coupling engagement with the spacer 78.

In other implementations, the spacer 78 may be secured to the threaded cup 16 via a taper connection. In still other implementations, the spacer 78 may be eliminated from the humeral implant assembly 99, and the liner 80 may be directly secured to the threaded cup 16, such as via either a taper connection or a C-clip, for example. Thus, this disclosure is not intended to be limited to the exact implementations shown in Figures 9-12B.

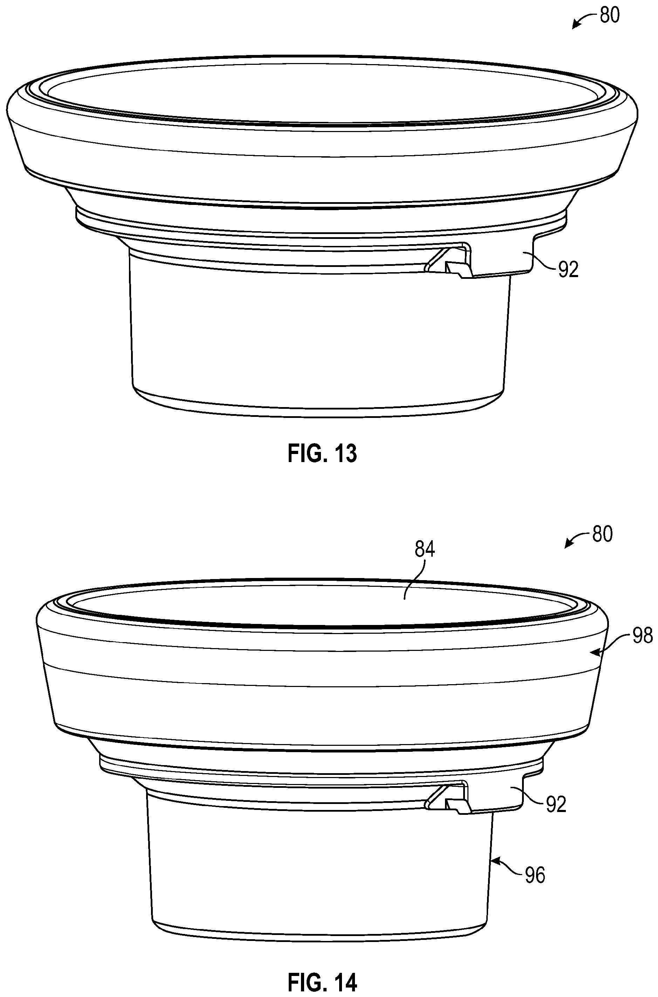

In an embodiment, the liner 80 is a metallic component (see Figure 13). In another embodiment, the liner 80 may include both a metallic portion 96 and a polymeric portion 98 (see Figure 14). The polymeric portion 98 may establish the concave articular surface 84 of the articular implant 82 and may be insert molded into a shell provided by the metallic portion 96.

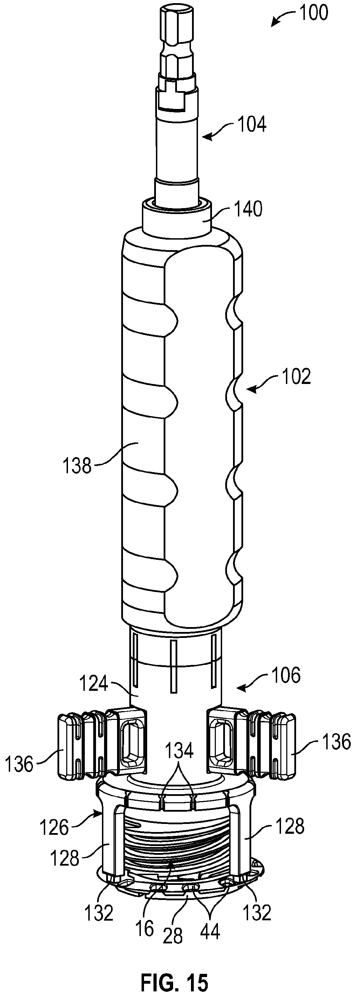

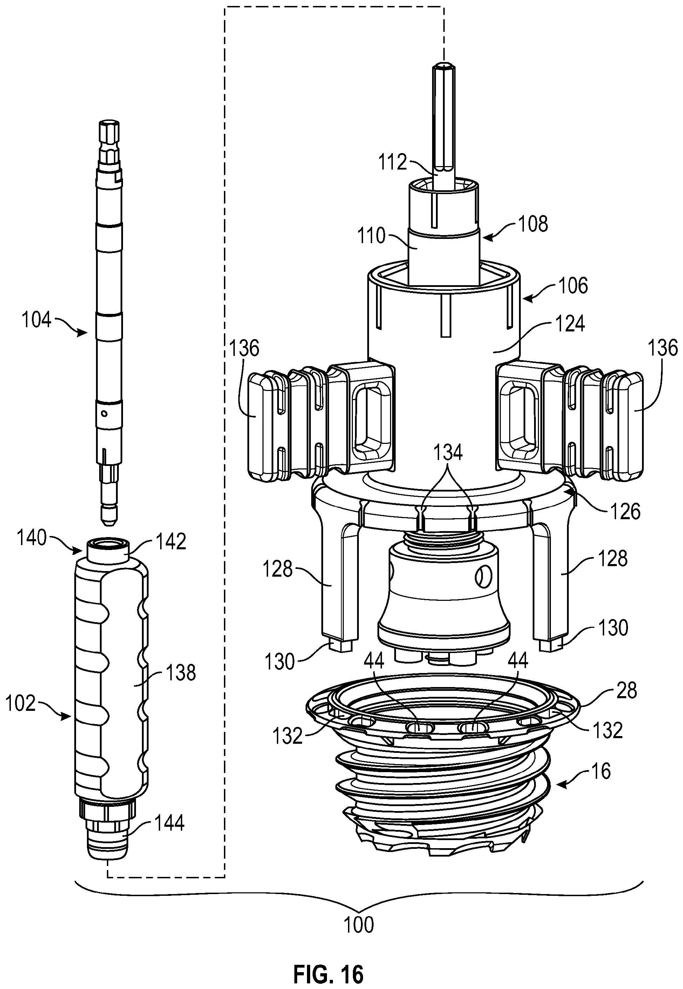

Figures 15, 16, 17, 18, and 19, with continued reference to Figures 1-14, illustrate an inserter system 100 for implanting the threaded cup 16 into the humerus 14. The inserter system 100 may include a handle 102, a drive shaft 104, a cage assembly 106, and a shaft assembly 108. Each of these components and their respective functions are further described below.

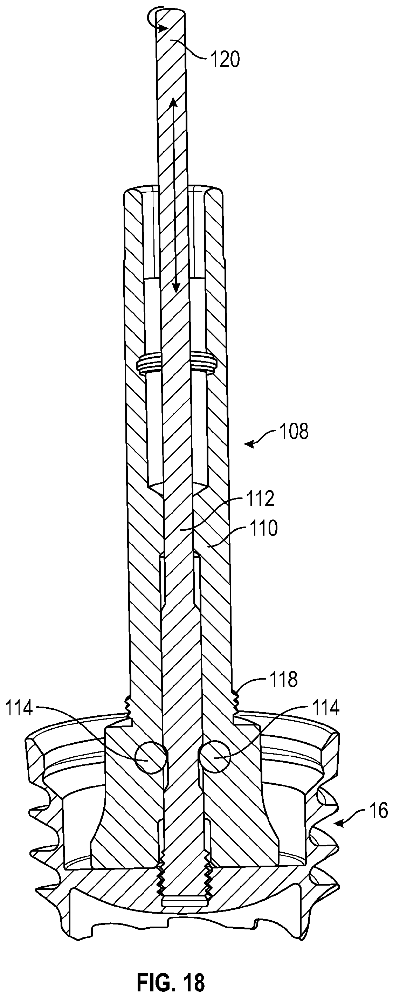

The shaft assembly 108 may include an outer shaft 110 and an inner shaft 112. The inner shaft 112 may be accommodated within a bore of the outer shaft 110 and may be secured in place relative to the outer shaft 110 by a pair of capture pins 114 (see Figure 18).

The inner shaft 112 may include a threaded distal tip 116 that may be screwed into the engagement opening 50 of the threaded cup 16 for attaching the inserter system 100 to the threaded cup 16. A proximal tip 120 of the inner shaft 112 may include a hex tip design or any other connection suitable for attaching a peripheral component (e.g., a knob) to the inner shaft 112 for more easily screwing the inner shaft 112 into the threaded cup 16.

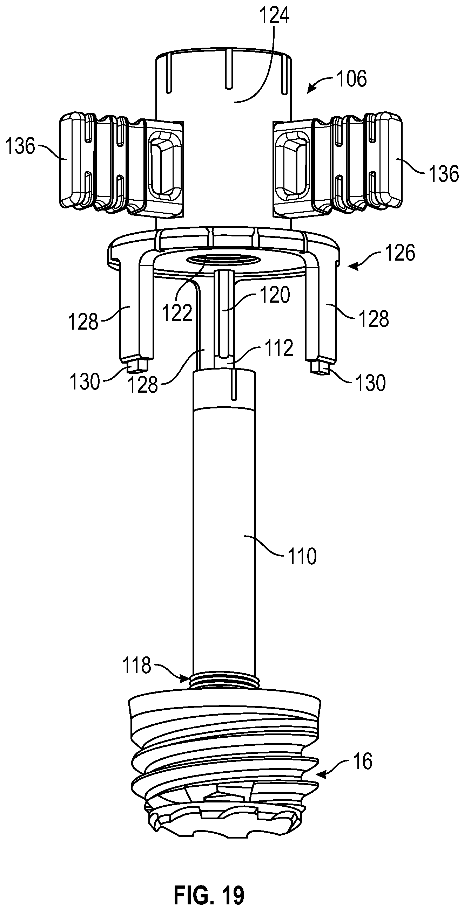

The outer shaft 110 may include an outer thread 118 for securing the shaft assembly 108 to the cage assembly 106. The outer thread 118 may be accommodated within a central threaded opening 122 of the cage assembly 106 (see Figure 19). The outer thread 118 and the central threaded opening 122 cooperate to maintain the threaded cup 16 recessed within the cage assembly 106 prior to insertion of the threaded cup 16 into bone.

The cage assembly 106 may include a proximal portion 124 that accommodates portions of the shaft assembly 108 and a distal portion 126 designed to interface with the flange 28 of the threaded cup 16. For example, the distal portion 126 may include a plurality of mounting legs 128 that each include a tapered tooth 130 sized to engage a tapered slot 132 that may be formed through the flange 28. The tapered slots 132 are additional openings that are separate from the suture eyelets 44. Sutures (not shown) that are accommodated within the suture eyelets 44 may be routed through the flange 28, then through slots 134 formed in the distal portion 126, and then may be wrapped around one or more suture wrap blocks 136 that can be attached to the proximal portion 124 of the cage assembly 106.

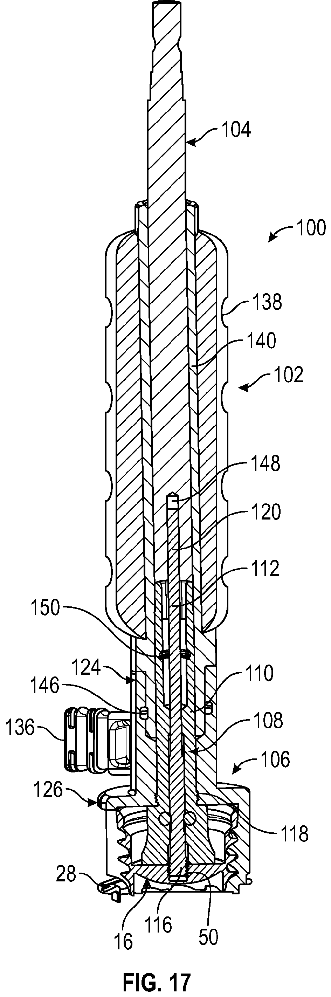

The handle 102 may include an outer grip 138 and an inner tube 140 that includes a proximal impaction face 142 and a distal connector 144. The distal connector 144 may be connected to the proximal portion 124 of the cage assembly 106, such as via a spring seal 146 (see Figure 17), for example. The proximal impaction face 142 may be exposed outside of the outer grip 138 and may be used for impacting the flange 28 onto the humerus 14. For example, the proximal impaction face 142 may be impacted by a surgical mallet for driving the anti-rotation tabs 35 of the flange 28 into the humerus 14, thereby securing the flange 28 in proper position at the cortical rim 40.

The drive shaft 104 may be inserted through the inner tube 140 of the handle 102 for connection to the inner shaft 112 of the shaft assembly 108. The drive shaft 104 may include a cannulation 148 for accommodating portions of the inner shaft 112. The drive shaft 104 may connect to the inner shaft 112 using another spring seal 150 (see Figure 17), for example.

After unscrewing the outer shaft 110 of the shaft assembly 108 from the cage assembly 106, the drive shaft 104 may be rotated in order to drive the threaded cup 16 into the humerus 14. The threaded cup 16 is screwed through the opening of the flange 28 as the flange 28 is being held against the cortical rim 40 by the distal portion 126 of the cage assembly 106. The various subcomponents of the inserter system 100 may then be removed from the threaded cup 16 and flange 28 to complete the procedure.

The exemplary arthroplasty implant systems of this disclosure employ modular stemless implants capable of establishing a convertible platform for interfacing with articular implants. The stemless implants may be configured as threaded cups that incorporate a modular flange (e.g., a circumferential ring/trunnion) that is adapted to rest atop the cortical rim of a resected bone in order to provide additional fixation support and load bone more favorably when implanted. The stemless implants may further provide inlaid designs that allow for inlay reverse prothesis configurations.

Although the different non-limiting embodiments are illustrated as having specific components or steps, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should further be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope and content of this disclosure.

## Claims

1. A humeral implant assembly (12) for an arthroplasty implant system (10), comprising:
an articular implant (18A, 18B); and
a modular stemless implant adapted to establish a convertible platform for receiving the articular implant (18A, 18B);
wherein the modular stemless implant includes a thread (36) configured to engage a bone and a flange (28) sized to engage a cortical rim (40) of the bone,
**characterized in that**
the flange (28) includes a plurality of suture eyelets (44) that are each configured to receive a thread-like material.

2. The assembly as recited in claim 1, wherein the articular implant (18A, 18B) includes a spacer (78) that is coupled to the stemless implant and a liner (80) that is coupled to the spacer (78), and further wherein the liner (80) includes a concave articular surface (84), and optionally wherein the spacer (78) is coupled to the stemless implant by a C-clip (86).

3. The assembly as recited in claim 1 or 2, wherein the modular stemless implant is comprised of a polyether ether ketone (PEEK) material.

4. The assembly as recited in any preceding claim, wherein the modular stemless implant includes a receiving cavity (32) adapted to receive the articular implant (18A, 18B), and further wherein the receiving cavity (32) extends inwardly from a rim to a floor (34) of a rounded base (30) of the modular stemless implant.

5. The assembly as recited in any preceding claim, wherein the thread (36) is circumferentially disposed about a radially outer surface (38) of a cylindrical shaped body (26) of the modular stemless implant, and optionally wherein the flange (28) is removably connectable to the cylindrical shaped body (26).

6. The assembly as recited in claim 5, wherein an outer diameter of the flange (28) is greater than an outer diameter of the cylindrical shaped body (26) at a tip of the thread (36), and optionally wherein the cylindrical shaped body (26) includes a plurality of pockets (54) adapted to facilitate bony ingrowth.

7. An arthroplasty implant system (10), comprising an assembly according to any of the previous claims:
the modular stemless implant being a two-piece modular threaded cup (16) that includes a cylindrical shaped body (26), a thread (36) provided on the cylindrical shaped body (26), and a flange (28) removably connectable to the cylindrical shaped body (26),
wherein the thread (36) is configured to engage a bone and the flange (28) is sized to engage a cortical rim (40) of the bone.

8. The system as recited in claim 7, wherein the flange (28) is a separate component from the cylindrical shaped body (26) to establish the two-piece modular design of the threaded cup (16).

9. The system as recited in claim 7 or 8, wherein the threaded cup (16) embodies an inlay design that establishes a convertible platform for receiving an articular implant (18A, 18B).

10. The system as recited in any of claims 7 to 9, wherein the flange (28) is provided on a first side of the cylindrical shaped body (26) and a rounded base (30) is provided on a second side of the cylindrical shaped body (26).

11. The system as recited in claim 10, wherein a receiving cavity extends inwardly from a rim of the cylindrical shaped body (26) to a floor (34) of the rounded base (30), and further wherein the floor (34) establishes an inner surface of the rounded base (30), and optionally comprising at least one engagement opening (50) formed through the rounded base (30).

12. The system as recited in any of claims 7 to 11, wherein an outer diameter of the flange (28) is greater than an outer diameter of the cylindrical shaped body (26) at a tip of the thread (36).

13. The system as recited in any of claims 7 to 12, comprising an inserter system that includes a drive shaft (104) configured to engage the cylindrical shaped body (26) and a cage assembly (106) configured to engage the flange (28), and optionally wherein the drive shaft (104) includes an inner shaft (112) having a threaded distal tip for engaging an engagement opening (50) of the cylindrical shaped body (26), and the cage assembly (106) includes a mounting leg (128) having a tapered tooth (130) configured to engage a tapered slot (132) of the flange (28).
